(19) Europäisches Patentamt
European Patent Office
Office européen des brevets

(11) Publication number: **0 453 021 A1**

(12) **EUROPEAN PATENT APPLICATION**

(21) Application number: **91200831.5**

(22) Date of filing: **10.04.91**

(51) Int. Cl.[5]: **C07C 45/53**, C07C 29/132, C07C 45/33, C07C 29/50, C07C 49/385, C07C 35/02, C07C 409/14

(30) Priority: **14.04.90 NL 9000893**

(43) Date of publication of application: **23.10.91 Bulletin 91/43**

(84) Designated Contracting States: **AT BE CH DE DK ES FR GB GR IT LI NL SE**

(71) Applicant: **STAMICARBON B.V.**
**Mijnweg 1**
**NL-6167 AC Geleen(NL)**

(72) Inventor: **Baur, Henricus Anna Christiaan**
**Rosslag 1**
**NL-6049 BE Herten(NL)**
Inventor: **Kragten, Ubaldus Franciscus**
**Beijensweide 15**
**NL-6191 EK Beek (L.)(NL)**
Inventor: **Wolvers, Wilhelmus Petrus**
**Godfried, Bomansstraat 35**
**NL-6372 KR Landgraaf(NL)**

(54) **Process for preparing a cycloalkanone and/or cycloalkanol.**

(57) The invention relates to a process for preparing a ketone and/or alcohol by oxidizing a cyclic hydrocarbon having at least 13 C atoms with oxygen to form a hydroperoxide, followed by a decomposition of the hydroperoxide in the presence of an organic metal complex, the decomposition being carried out in the presence of a phthalocyanine or porphyrine metal complex immobilized on a carrier.

EP 0 453 021 A1

The invention relates to a process for preparing a ketone and/or alcohol by oxidizing a cyclic hydrocarbon having at least 13 C atoms with oxygen to form a hydroperoxide, followed by a decomposition, in the presence of an organic metal complex, of the hydroperoxide formed.

In Liebigs Ann. Chem. 757; pp. 109-120 (1972) Kropf and Knaack describe an investigation into the autooxidation of phenylcycloheptane (a cyclic hydrocarbon with 13 C-atoms) with a Cu-phthalocyanine as catalyst to form the corresponding peroxide. Some mention is made of the formation of a cyclic ketone, but the publication is substantially only aimed at the formation of hydroperoxide. Any information about the selectivity of the reaction is lacking.

In DE-A-1.940.051 a process is described for oxidizing aromatic hydrocarbons with condensed ring systems, such as anthracene or fluorene, to form the corresponding oxidation product (such as anthraquinone). In that process, the catalyst used is a salt of a heavy metal. No mention is made of the target-oriented formation of the intermediate hydroperoxide.

It has been found that, for the selective preparation of a ketone and/or alcohol by the oxidation of a cyclic hydrocarbon with at least 13 C atoms, a better result is obtained if the formation of the hydroperoxide and the decomposition thereof are performed as separate process steps. Thus an optimum preparation of the hydroperoxide can be linked with an optimum conversion thereof into the corresponding ketone and/or alcohol.

In so far as an organic metal complex is applied in the processes described in literature, the complex is used in a soluble form, i.e. the complex is present in the oxidizing liquid in a homogeneously dissolved state. As a consequence, the complex must necessarily be separated from the reaction mixture separately, for instance by distillation. In addition to the fact that, owing to such thermal effect, (a part of) the thermally sensitive complex may be lost, the complex must also be recovered from the distillation product before it can be used again in the process.

So there is a need for a process in which the above and other disadvantages of the known processes are met. This is done according to the invention in that the decomposition of the hydroperoxide is carried out in the presence of a phthalocyanine and/or porphyrine metal complex immobilized on a carrier.

Owing to the immobilization on the carrier, a catalyst is obtained which can easily be separated off from the reaction phase and which combines a surprisingly good, sustained and stable activity with a good selectivity to the ketone and/or alcohol.

The starting material that may be used for the process according to the invention may be any substituted or non-substituted cyclic hydrocarbon with at least 13 C atoms. The oxidation of such a compound is such that the hydroperoxide is formed on a secondary carbon atom of a hydrocarbon having secondary and possibly primary carbon atoms. The hydroperoxide is formed on a tertiary carbon atom if (in additon to any secondary and primary carbon atoms) such an atom is present in the compound to be oxidized. In the case of a secondary hydroperoxide, this hydroperoxide is converted during the composition to form a ketone and/or an alcohol; in the case of a tertiary hydroperoxide conversion into an alcohol takes place.

In Beilstein (V-Hauptwerk) a great many cyclic hydrocarbons are given suitable for the invention. Besides the following non-limitative enumeration, reference be made thereto.

Cyclic hydrocarbons that can, according to a process of the invention, be converted into a ketone and/or alcohol have at least 13 carbon atoms. Preferably they have 13-40 carbon atoms, in particular 13-30. Examples of applicable cyclic hydrocarbons are:

A. 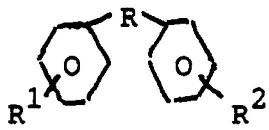

R = a direct bond or (CH2)n
n = 1,2
$R^1$, $R^2$ = hydrogen, methyl, ethyl, isopropyl, isobutyl, tert.-butyl, etc., aryl;

B. 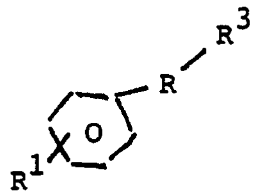

R = methylene, cycloalkylene;
$R^1$ = hydrogen, methyl, ethyl isopropyl, isobutyl, tert.-butyl, etc., aryl;
$R^3$ = cycloalkyl, methyl, hydrogen;

C. 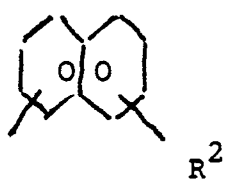

$R^1$, $R^2$ = hydrogen, methyl, ethyl, isopropyl, isobutyl, tert.-butyl, etc., aryl;

D. 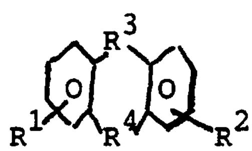

$R^3$ = a direct bond or $(CH_2)_n$
n = 1,2
$R^4$ = a direct bond or $(CH_2)_n$
m = 1,2; preferably $R^3=R^4$
$R^1$, $R^2$ = hydrogen, methyl, ethyl, isopropyl, isobutyl, tert.-butyl, aryl, etc.;

E. 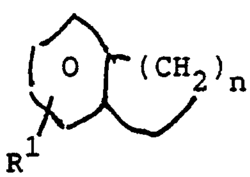

n = 2-12
$R^1$ = hydrogen, methyl, isopropyl, isobutyl, tert.-butyl, aryl, etc.;

F. 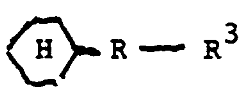

R = methylene, ethylene, cycloalkylene;
$R^3$ = hydrogen, methyl, cycloalkyl.

$R^1$ and/or $R^2$ may represent one or more substituents to the ring.

Examples of such compounds are:

For group A: - diphenylmethane
- diphenylethane

- 2-ethyl-1,1'-biphenyl
- 3-ethyl-1,1'-biphenyl
- 2-isopropyl-1,1'-biphenyl

For group B:
- phenylcyclododecane
- phenylcycloheptane
- 2-methyl-1-cyclohexyl-benzene
- 3-methyl-1-cyclohexyl-benzene
- 4-tert. butyl-1-cycloheptyl-benzene

For group C:
- 2-isopropylnaphthalene
- 1-isobutylnaphthalene
- 2-isobutylnaphthalene
- 1-ethyl-4-methylnaphthalene

For group D:
- fluorene
- g-methylfluorene
- 9,10-dihydrophenanthrene
- 1-methylfluorene
- 1,8-dimethylfluorene

For group E:
- 1,2,3,4,-tetrahydro-6-phenyl-naphthalene

For group F:
- 1,2-dicyclohexyl-ethane
- 1,1-dicyclohexyl-methane

Phthalocyanine and porphyrine metal complexes that can be used in a process according to the invention are known per se. In this connection reference may be made, for instance, to the article by J. Manassen in Cat. Rev. Sci. Eng. 9(2), 223-43 (1974). The preparation of such complexes is described, inter alia, by B. Berezin in Coordination Compounds of Porphyrins and Phthalocyanines, Wiley N.Y. 1981. The starting material for the said complexes is a porphyrine or a phthalocyanine, which may be substituted. The structure formulas of these compounds are as follows:

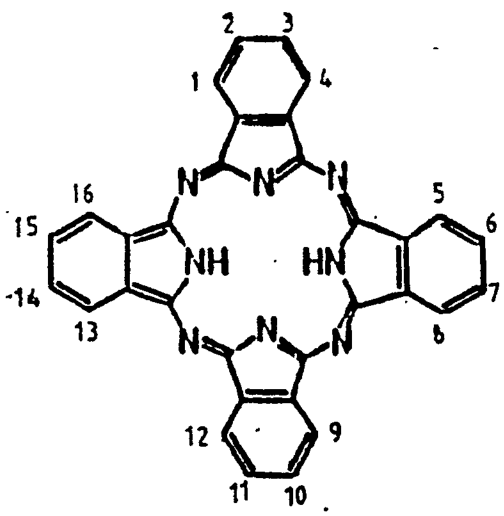

Phthalocyanine

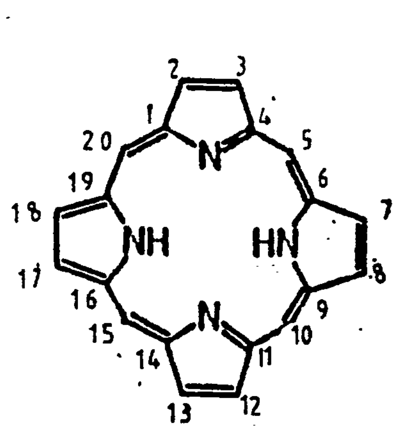

Porphyrine

Phthalocyanines may be substituted at the indicated positions 1-16, porphyrines at the indicated positions 1-20, except for the positions 1, 4, 6, 9, 11, 14, 16 and 19. The substituents used may be:

a. H, F, Cl, Br and I,
b. alkyl groups, whether or not substituted or functionalized,
c. alkenyl groups, whether or not substituted or functionalized,
d. phenyl groups, whether or not substituted or functionalized,
e. amines, sulphonic acids, carboxylic acids, aldehydes and derivatives thereof.

The complexation of a porphyrine can for example be effected by introducing the porphyrine into DMF (dimethylformamide) and adding, under reflux, the metal to be incorporated as metal chloride. Phthalocyanines can be synthesized by starting from molecular fragments such as phthalonitrile,

phthalimide and phthalic anhydride. The metal sources used may be metal chlorides. In some cases urea is used as nitrogen donor and ammonium molybdate as catalyst.

The metals most suited for applying the process are Co, Mn, Cr, Fe and/or V, but in principle any transition metal capable of forming organometallic complexes with said products will qualify.

Mixtures of the said metals can be used also.

According to the invention, the organometallic complexes are bonded to (immobilized on) a carrier. The carrier may be any material that can be used in effecting a bond with the complex. In this connection an ionogenic, a coordinative, as well as a covalent bond may be thought of. In order to obtain an effective bond, the substituted groups of the organometallic complexes must be such as to make such a bond possible. This can be obtained, for instance, by using complexes containing one or more sulphonic acid, amino or carboxylic acid groups or combinations thereof.

The carrier to be used may be of an inorganic as well as an organic polymeric nature. The carrier, too, must be provided with one or more groups capable of immobilizing the organometallic complex. Among the requirements which the carrier must comply with is that the carrier must have enough reactive groups to enable it to acquire an acceptable degree of loading. The suitable reactive groups are, for instance, COOH, NHR, OH, $SO_3H$, Cl, Br, I, but also phenyl and associated groups. Further, the material must not dissolve in one of the components present in the process stream, it must be inert to the reactions that take place and it must be mechanically stable.

As carrier, inorganic carriers, such as alumina, TiO2, SiO2 or organic carriers, such as polystyrene, ethylenevinylacetate copolymer, acid or anhydride-modified polyethylene, qualify.

When, for instance, silica is used as carrier material, the bond may be effected, for instance, by starting from a phthalocyanine or porphyrine with one or more substituents containing halogen(compounds). The starting material is heated for some time in pyridine, together with the silica. The solid is subsequently filtered off, washed and dried.

When, for instance, polystyrene is used as carrier material, the bonding may be effected, for instance, by starting from a phthalocyanine or porphyrine with one or more substituents containing COOH groups. By applying a Friedel-Crafts reaction it can be bonded to polystyrene.

In the process according to the invention the oxidation of the cyclic hydrocarbon in the liquid phase is carried out with, for instance, air, pure oxygen or a mixture of oxygen and inert gas at temperatures of 70-200 °C during $\frac{1}{2}$-24 hr. In the process 1-30%, for instance, of the hydrocarbon is converted in order to obtain a maximum yield of the intermediate hydroperoxide. The pressure in this oxidation process is not critical and is, for practical reasons, generally between 1 and 50 bar. The reaction can be carried out in the cyclic hydrocarbon as such, as well as by applying a solvent. The solvent used may be, for instance, benzene or toluene.

The oxidation of the cyclic hydrocarbon is carried out preferably in the absence of substances promoting the decomposition of the hydroperoxide formed, such as compounds of transition metals, and for this reason preference is given in this oxidation process to the use of a reactor with an inert inner wall, for instance an inner wall of passivated steel, aluminium, glass, enamel and similar materials. If yet the use of an oxidizing catalyst is desired, the amount of transition metal should preferably be very small, for instance in the order of 1-10 parts by weight per million. The oxidizing catalyst used may consist of compounds of, for instance, cobalt, chromium, manganese, iron, nickel, copper or mixtures thereof. The organometallic complexes described are suited also.

The decomposition of the hydroperoxide in the oxidation mixture is effected by means of the immobilized metal complexes based on phthalocyanine and/or porphyrine. The decomposition catalyst can be used in various ways. As it is applied on a carrier, slurry reactors as well as, for instance, packed beds can be used to effect the conversion of the hydroperoxide. The heat of reaction released in the decomposition must be adequately collected and carried off to guarantee a proper process temperature control. This can be done well, particularly when slurry reactors are used. During the decomposition the desired temperature can then, for instance, be maintained by refluxing at least a part of the heat to be carried off. No recirculation of evaporated products will then be required, which has a somewhat favourable effect on the yield of desired product.

The amount of complex to be used is, for instance, 1-250 ppm metal, calculated on the oxidation mixture. Preference is given to the use of 5-150 ppm metal.

The temperature during the decomposition is generally within the range of 25-150 °C. The pressure chosen in the decomposition is usually slightly lower than in the oxidation. The decomposition is preferably carried out in the presence of oxygen. This causes the yield of the ketone and/or alcohol to be improved. The necessary residence time for the reaction mixture can be determined easily by analysis of any not decomposed peroxide. Generally a residence time between 5 and 300 minutes will qualify, preferably the

residence time will be chosen from 10-120 minutes.

Before the decomposition of the hydroperoxide in the oxidation mixture, the oxidation mixture can be treated, if so desired, with water or with an aqueous alkali metal hydroxide or alkali metal carbonate solution for the removal and/or neutralization of the acids formed in the oxidation, for instance to a pH of the aqueous phase of 8-13. The non-converted cyclic hydrocarbon can (partially) be removed from the oxidation mixture, for instance by applying a distillation or flash evaporation process.

The reaction mixture obtained in the decomposition of the hydroperoxide can be further processed by subjecting the organic phase, after washing with water if so desired, to a distillation treatment while recovering cyclic hydrocarbon, which is to be returned, and the ketone and/or alcohol.

The invention will be further elucidated by means of the following examples.

Example I

To 35 ml DMF (dimethylformamide) 5 mmoles dicyclohexylcarbodiimide was added at room temperature. Subsequently, 0.5 mmole Co-tetrasulphonic acid phthalocyanine was added. This mixture was stirred for one hour at room temperature. After that, 1 gramme silica modified with 3-aminopropyltrimethoxysilane (BET-surface = 390 $m^2/g$; particle size 0.5-1 mm; N (nitrogen content) = 2.4% (wt); C (carbon content) = 6.5% (wt)) was added. This mixture was stirred for 24 hours at room temperature, filtered and washed with ethanol and dichloromethane and dried at 60 °C. The cobalt content was 0.56% (wt).

Example II

Example I was repeated with Fe and V-tetrasulphonic acid phthalocyanine as compounds to be bonded. The metal content of the products was respectively 0.38% (wt) iron and 0.42% (wt) vanadium.

Example III

In 200 ml DMSO (dimethylsulfoxide), 2.0 grammes Ti-tetraaminephthalocyanine was dissolved. To this solution was added 1.5 grammes silica modified with 2-(4-chlorosulphonylphenyl)ethyltrimethoxysilane (BET = 540 $m^2$; 0.5-1 mm; C = 14.8% (wt); S (sulphur content) = 4.3% (wt)).

After stirring for one day, the loaded silica was filtered off and washed with DMSO and acetone. The titanium weight percentage on the silica was 0.37%.

Example IV

Example III was repeated with Fe and Co-tetraaminephthalo-cyanine as compounds to be bonded. The iron and cobalt contents were respectively 0.36 and 0.25% (wt).

Example V

To a solution of cobalttetrabromophthalocyanine in pyridine was added 10 grammes silica (particles with a diameter of 3 mm and a BET surface of about 60 $m^2$/gramme). The suspension was stirred for 6 hours at 70 °C. After cooling, the suspension was filtered and washed with methanol and chloroform, and dried. The reaction product was analysed for its metal content (0.05% (wt)).

Example VI

Example V was repeated with Co-monochlorophthalocyanine (metal content 0.08% (wt)) as compound to be bonded.

Example VII

Example V was repeated with (4(3-bromo-1-propoxy)-phenyl), 10,15,20-tritolylporphyrine as compound to be bonded. After bonding, Cr, Co, V, Mn and Fe were incorporated as metals using the DMF method. All these products were analysed for their metal content.

**Results:**

| | |
|---|---|
| Cr: 0.09% (wt) | Mn: 0.06% (wt) |
| Co: 0.05% (wt) | Fe: 0.06% (wt) |
| | V : 0.04% (wt) |

Example VIII

To 20 grammes of a fluorene oxidation mixture, containing 3% (wt) 9-hydroperoxyfluorene, 1% (wt) fluorenol and 1% (wt) fluorenone in fluorene, dissolved in 200 grammes benzene, was added such an amount of the catalyst mentioned in example I that the resulting metal concentration was 50 ppm. At a temperature of 65° C the peroxide decomposed completely within 60 minutes. The velocity constant k (based on a first order decomposition) was 0.09 $min^{-1}$. The selectivity to fluorenol and fluorenone was 98.5%. The turnover figure (defined as moles converted peroxide per mole metal) that could be reached in re-use was higher than 3500.

Comparative experiment A

Example VIII was repeated with Co-phthalocyanine as catalyst.

The catalyst dissolved partly in the oxidation mixture. The k was 0.01 $min^{-1}$, the selectivity 95%. The turnover figure in re-use was lower than 500.

Comparative experiment B

Example VIII was repeated with Co-acetate as catalyst. The k was 0.02 $min^{-1}$ . The catalyst, however, precipitated from the reaction mixture and could not be used again. The selectivity was 95%.

Example IX

To 20 grammes of an oxidation mixture of triphenylmethane containing 7% (wt) triphenylmethanehydroperoxide and 2% (wt) triphenylmethanol in triphenolmethane, dissolved in 200 grammes benzene, was added such an amount of the Fe catalyst mentioned in example II that the resulting metal concentration was 60 ppm. At a temperature of 65° C the peroxide decomposed completely within 60 min. The k was 0.15 $min^{-1}$, the selectivity to triphenylmethanol 94.5%. The turnover figure that could be reached in re-use was higher than 2200.

Example X

Example IX was repeated with the Fe catalyst of example IV.

The k was 0.16 $min^{-1}$ and the selectivity to triphenylmethanol was 94.5%.

Example XI

Example IX was repeated with the Cr catalyst of example VII.

The k was 0.04 $min^{-1}$ and the selectivity to the triphenylmethanol was 96.4%.

Example XII

To 20 ml thionylchloride 4 mmoles Co-tetracarboxyphthalocyanine was added. This mixture was stirred at room temperature for a few hours in an $N_2$ atmosphere. Subsequently, a suspension of 1,1,2,2-tetrachloroethane and polystyrene (20 grammes, 3% wt vinylbenzene, 22-50 mesh, macroporous 8000 nm) was added. The reaction mixture was heated to 135° C and the excess of SOC12 was distilled off. The reactor mass was cooled to 12° C, upon which 6 grammes $AlCl_3$ was added to it. The reaction mixture was stirred for 20 hours. After that the solid was filtered, washed with 1,1,2,2-tetrachloroethane, methanol, basic water and 1 N HCL. The resulting solid was dried at 50° C. The product was analysed for its metal content.

(Cobalt content was 0.43% (wt)).

Example XIII

Example IX was repeated with Mn-octacarboxyphthalocyanine as the compound to be bonded. (Manganese content was 0.53% (wt)).

Example XIV

Example IX was repeated with the 5,10,15,20-tetra-(4-carboxyphenyl)porphyrineas the compound to be bonded. The metals incorporated after bonding by means of the DMF method were Cr, Co, Mn, Cu and Fe.

All these products were analysed for their metal content.

**Metal content:**

Cr = 1.9% (wt)    Mn = 0.61% (wt)

Co = 0.49% (wt)    Cu = 0.50% (wt)

Fe = 0.64% (wt)

Example XV

To an oxidation mixture of 17% (wt) 2-isopropyl-hydroperoxy-naphthalene containing 2-isopropylnaphthalene such an amount of the Co catalyst mentioned in example XII was added that the resulting metal concentration was 100 ppm. The peroxide decomposed completely within 60 minutes at a temperature of 85° C.

The velocity constant k was 0.04 $min^{-1}$ . The selectivity to 2-isopropylhydroxynaphthalene was 96.2%. The turnover number that could be reached in re-use was higher than 2800.

Example XVI

Example XV was repeated with the Mn catalyst of example XIII.

The k was 0.003 $min^{-1}$ . The selectivity was 92.5%.

Example XVII

To an oxidation mixture of 2-ethyl-7-methylnaphthalene, containing 5% (wt) 2-ethyl(1'-hydroperoxy)-7-methyl-napthalene, 1.5% (wt) 2-ethyl(1'-hydroxy)-7-methylnaphthalene and 0.8% (wt) 2-ethyl(1'-one)-7-methylnaphthalene in 2-ethyl-7-methyl-naphthalene such an amount of the Cr catalyst mentioned in example XIV was added that the resulting metal concentration was 85 ppm. The peroxide decomposed completely within 60 minutes at a temperature of 95° C. The k was 0.04 $min^{-1}$ . The selectivity to the alcohol and to the ketone was 98.2 %. The catalyst could be re-used a plurality of times.

Example XVIII

Example XVII was repeated with the Fe catalyst of example XIV.

The k was 0.12 $min^{-1}$. The selectivity to the alcohol and to the ketone was 105%.

Example XIX

Example XVIII was repeated, air being passed through the solution, too. As a result, the selectivity to the alcohol and to the ketone rose to 105%.

Comparative example C

Example XVII was repeated with ditertiarybutylchromate as catalyst. The k was 0.003 $min^{-1}$. The

selectivity was 96.5%. After the experiment, the catalyst was found to be de-activated; newly added peroxide was found not to be capable of being decomposed.

## Claims

1. Process for preparing a ketone and/or alcohol by oxidizing a cyclic hydrocarbon having at least 13 C atoms with oxygen to form a hydroperoxide, followed by a decomposition, in the presence of an organic metal complex, of the hydroperoxide formed, characterized in that the decomposition is carried out in the presence of a phthalocyanine or porphyrine metal complex immobilized on a carrier.

2. Process according to claim 1, characterized in that the metal in the complex is Co, Mn, Cr, Fe and/or V.

3. Process according to any one of claims 1-2, characterized in that the carrier is of an inorganic nature.

4. Process according to any one of claims 1-2, characterized in that the carrier is of an organic polymeric nature.

5. Process according to any one of claims 1-4, characterized in that the preparation is carried out in a slurry reactor.

6. Process according to any one of claims 1-5, characterized in that 10-150 ppm metal complex is applied calculated on the metal in the complex and on the oxidation mixture.

7. Process according to any one of claims 1-6, characterized in that the decomposition of the hydroperoxide is carried out in the presence of oxygen.

European Patent Office

# EUROPEAN SEARCH REPORT

Application Number

**EP 91 20 0831**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 367 326 (STAMICARBON B.V.)<br>* Claims * | 1-7 | C 07 C 45/53<br>C 07 C 29/132<br>C 07 C 45/33<br>C 07 C 29/50<br>C 07 C 49/385<br>C 07 C 35/02<br>C 07 C 409/14 |
| X,Y | HOMOGENEOUS HETEROG. CATAL., PROC. INT. SYMP. RELAT. HOMOGENEOUS HETEROG. CATAL., 5TH, 1986, pages 343-365; B.V. ROMANOVSKY: "Catalysis by oxide-supported porphyrine and phthalocyanine complexes"<br>* Pages 358-359 * | 1-3 | |
| Y | CHEMICAL ABSTRACTS, vol. 104, 1986, page 629, column 1, abstract no. 168029f, Columbus, Ohio, US; & JP-A-60 126 237 (SUMITOMO CHEMICAL CO., LTD) 05-07-1985<br>* Abstract * | 1,2 | |
| Y | CHEMICAL ABSTRACTS, vol. 107, 1987, page 661, column 1, abstract no. 77359j, Columbus, Ohio, US; T.V. KOROL'KOVA et al.: "Liquid-phase oxidation of cumene on iron(II) phthalocyanine fixed in a sodium-Y zeolite", & NEFTEKHIMIYA 1986, 26(4), 546-8<br>* Abstract * | 1-3 | **TECHNICAL FIELDS SEARCHED (Int. Cl.5)** |
| Y | FR-A-1 530 986 (ICI LTD)<br>* Page 1, columns 1,2; page 2, column 1, claims * | 1,2 | C 07 C 45/00<br>C 07 C 29/00 |
| Y | US-A-3 816 548 (R.H. WILLIAMS et al.)<br>* Column 3, claims * | 1,2 | |
| Y | ANGEWANDTE CHEMIE, vol. 19, no. 11, 1980, pages 909-910; D. MANSUY et al.: "Metalloporphyrin-catalyzed hydroxylation of cyclohexane by alkyl hydroperoxides: Pronounced efficiency of iron-porphyrins"<br>* Page 909, column 2 * | 1,2 | |

The present search report has been drawn up for all claims

| Place of search | Date of completion of search | Examiner |
|---|---|---|
| The Hague | 26 June 91 | BONNEVALLE E.I.H. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document